# EUROPEAN PATENT SPECIFICATION

(11) **EP 4 228 584 B1**
(45) Date of publication and mention of the grant of the patent: **19.03.2025**
(21) Application number: 21797949.1
(22) Date of filing: 11.10.2021
(51) Int. Cl.: A61J 7/00, A61B 5/00, G16H 20/13

(54) **MEDICATION DELIVERY**
MEDIKAMENTENVERABREICHUNG
DISTRIBUTION DE MÉDICAMENTS

(30) Priority: 14.10.2020 SE 2051195
(43) Date of publication of application: 23.08.2023
(73) Proprietor: Dosell AB, 111 38 Stockholm (SE)
(72) Inventor: SJÖNELL, Göran, 181 46 Lidingö (SE)
(74) Representative: Rouse AB
(86) International application number: PCT/EP2021/078015
(87) International publication number: WO 2022/078943

(56) References cited:
- US-A1- 2007 038 330
- US-A1- 2017 185 745
- US-A1- 2018 165 421
- US-A1- 2019 156 945

## Description

### TECHNICAL FIELD

The present disclosure relates generally to systems and methods for medication delivery. Such a system is disclosed in the US2007038330.

### BACKGROUND

On the market, there are a number of different mechanized and automatic medication dispensing devices (also called e.g. automated medication dispensers, automatic pill dispensers or dosage devices). Such devices are configured to receive and house medication, and to output predefined doses of medication at certain points in time according to a selected schedule. The pharmaceuticals (i.e. the medication) are stored inside the medication dispensing device and are not accessible by the patient, except for the medication that is output for consumption by the patient at a certain point in time.

Such medication dispensing devices may store the different types of medication in different medication containers within the medication dispensing device, with one container for each type of medication. The medication dispensing device then dispenses a predefined number of predefined types of medication to the patient at a predefined time, directly from the different medication containers. This type of medication dispensing device is easy to load for a caregiver, since what is needed is simply to ensure that there is enough medication of the right type in the each medication container. However, it is not possible to double-check exactly what has been dispensed to the patient if there is a malfunction in such a medication dispensing device.

There are also medication dispensing devices, such as e.g. the automatic pill dispenser described in US4573606, that comprise a number of compartments, where each compartment stores a predefined dose of medication. Such medication dispensing devices are generally safer that the above described kind, but the process of loading each compartment with the right number of pills may be very cumbersome.

There are therefore also medication dispensing devices that deliver the medication in prepackaged medication packages, that normally come in rolls from a medication storage facility. All the medication to be taken at a certain time has in this case been prepackaged into each medication package. However, this requires the prepackaging and distribution of such medication packages, by the medication storage facility, before the loading of the medication packages into the medication dispensing device. Such medication dispensing devices are known from e.g. US9323897 and SE542287.

### PROBLEMS WITH THE PRIOR ART

A medication dispensing device is typically used to ascertain that the patient takes any prescribed medication at the prescribed time intervals and in the prescribed dose. However, since the medication is often taken in the home of the patient, there is no way of monitoring how the patient is affected by the medication. Some medication may have side-effects such as e.g. edema or dehydration, and there may also be undesirable effects by over- or undermedication. It would therefore be useful to be able to monitor the effects of the medication, and to adjust the medication based on these effects.

US2020/0185078 describes a method that may be used to adjust the medication dispensed by a medication dispensing device in which the medication is manually loaded into different containers or compartments. However, the method described in US2020/0185078 cannot be used to adjust the medication dispensed from a medication dispensing device that receives the medication in prepackaged medication packages. There is thus a need for improved systems and methods for medication delivery.

### SUMMARY

The invention is disclosed in the claims. The above described problem is addressed by the claimed system for medication delivery. The claimec system may comprise: a medication dispensing device, arranged to deliver a medication package containing a predefined dose of medication to a patient at a preset time; a medication distribution system, arranged to distribute the medication packages containing predefined doses of medication to the medication dispensing device; at least one sensing arrangement, arranged in the same premises as the medication dispensing device, and arranged to measure at least one patient specific parameter for said patient; and at least one processor, arranged to process the at least one patient specific parameter, and notify the medication distribution system if the at least one patient specific parameter is determined to deviate from an expected range. The medication distribution system may be arranged to, based on the result of the processing of the at least one patient specific parameter, adjust the dose of medication in the medication packages that are distributed to the medication dispensing device. Such a system allows the adjustment of the medication based on the effects of the medication, for a medication dispensing device that delivers the medication in medication packages.

In embodiments, the medication distribution system is arranged to receive input from at least one care provider who is responsible for the care of the patient regarding the adjustment of the dose of medication in the medication packages that are distributed to the medication dispensing device by the medication distribution system. This allows the physician who has prescribed the medication to adjust the dose based on the effects of the medication.

In embodiments, the system comprises a display which is arranged to display information about the result of the processing of the at least one patient specific parameter to the patient. Such a display may be arranged on the medication dispensing device, but it may also be integrated into a device that e.g. is wearable by the patient, such as e.g. a watch or a wristband. It is also possible to use the display on e.g. a smartphone. This may be a way to provide feedback to the patient about the effects of the medication.

In embodiments, the at least one sensing arrangement is arranged in a position where the patient is located when receiving medication from the medication dispensing device. This ensures that the at least one patient specific parameter is measured at least at the times that the patient receives medication.

In embodiments, the at least one sensing arrangement comprises a body weight measuring device. In embodiments, the body weight measuring device is arranged in the form of weight sensors provided on or in an object on which the weight of the patient regularly rests, such as e.g. a carpet or rug, a mattress, a chair, a sofa, an armchair, or a toilet seat. This ensures that the current weight of the patient is determined on a regular basis without any action being required from the patient. The body weight measuring device may be arranged to only determine the body weight if the system has determined that the patient is located on the body weight measuring device.

In embodiments, the at least one sensing arrangement is arranged to monitor any changes in body weight of the patient over time, e.g. by calculating a daily mean body weight and monitor the change in said daily mean body weight over time. In order for a determination of a daily mean body weight to be meaningful, the body weight should be measured a number of times each day, e.g. at least three times a day.

In embodiments, the at least one sensing arrangement comprises: a body weight sensor, a motion sensor, a temperature sensor, proximity sensor, a temperature sensor, a continence sensor, a breathing frequency sensor, a heart rate sensor, a blood pressure sensor, and/or a blood glucose sensor. All these sensors may provide information regarding how the patient is affected by the medication.

In embodiments, historical data from the at least one sensing arrangement is used for determining whether the at least one patient specific parameter deviates from the expected range, e.g. using a machine learning system, based on e.g. daily mean values and/or the pattern of parameter fluctuation over time. Machine learning is an efficient way of analyzing large quantities of data.

The above described problem is further addressed by the claimed method for medication delivery. The method may comprise: automatically delivering a medication package containing a predefined dose of medication to a patient at a preset time, using a medication dispensing device; automatically distributing the medication packages containing the predefined doses of medication to the medication dispensing device, using a medication distribution system; automatically measuring at least one patient specific parameter for said patient using at least one sensing arrangement; automatically processing, in at least one processor, the at least one patient specific parameter; automatically notifying the medication distribution system if the at least one patient specific parameter is determined to deviate from an expected range; and adjusting, based on the result of the processing of the at least one patient specific parameter, the dose of medication in the medication packages that are distributed by the medication distribution system to the medication dispensing device. Such a method allows the adjustment of the medication based on the effects of the medication, for a medication dispensing device that delivers the medication in medication packages.

In embodiments, the method further comprises receiving input from at least one care provider who is responsible for the care of the patient regarding the adjustment of the dose of medication in the medication packages that are distributed to the medication dispensing device by the medication distribution system. This allows the physician who has prescribed the medication to adjust the dose based on the effects of the medication.

In embodiments, the method further comprises displaying information about the result of the processing of the at least one patient specific parameter to the patient on a display. Such a display may be arranged on the medication dispensing device, but it may also be integrated into a device that e.g. is wearable by the patient, such as e.g. a watch or a wristband. It is also possible to use the display on e.g. a smartphone. This may be a way to provide feedback to the patient about the effects of the medication.

In embodiments, the method further comprises arranging the at least one sensing arrangement in a position where the patient is located when receiving medication from the medication dispensing device. This ensures that the at least one patient specific parameter is measured at least at the times that the patient receives medication.

In embodiments, the method further comprises arranging the at least one sensing arrangement to comprise a body weight measuring device. In embodiments, the method further comprises arranging the body weight measuring device in the form of weight sensors provided on or in an object on which the weight of the patient regularly rests, such as e.g. a carpet or rug, a mattress, a chair, a sofa, an armchair, or a toilet seat. This ensures that the current weight of the patient is determined on a regular basis without any action being required from the patient. The body weight measuring device may be arranged to only determine the body weight if the system has determined that the patient is located on the body weight measuring device.

In embodiments, the method further comprises automatically monitoring any changes in body weight of the patient over time, e.g. by calculating a daily mean body weight and monitor the change in said daily mean body weight over time. In order for a determination of a daily mean body weight to be meaningful, the body weight should be measured a number of times each day, e.g. at least three times a day.

In embodiments, the method further comprises arranging the at least one sensing arrangement to comprise: a body weight sensor, a motion sensor, a temperature sensor, proximity sensor, a temperature sensor, a continence sensor, a breathing frequency sensor, a heart rate sensor, a blood pressure sensor, and/or a blood glucose sensor. All these sensors may provide information regarding how the patient is affected by the medication.

In embodiments, the processing comprises using historical data from the at least one sensing arrangement for determining whether the at least one patient specific parameter deviates from the expected range, e.g. using a machine learning system, based on e.g. daily mean values and/or the pattern of parameter fluctuation over time. Machine learning is an efficient way of analyzing large quantities of data.

The term "medication" covers any type of pharmaceuticals, including combinations of different types of pills.

The term "dose" covers any number of pills, of one or more different types of pharmaceuticals.

The at least one processing device may be one processing device, or a number of processing devices between which signals are transmitted. Some processing may e.g. take place in one processing device, and signals may then be transmitted to one or more other processing devices for further processing.

The scope of the invention is defined by the claims, which are incorporated into this section by reference. A more complete understanding of embodiments of the invention will be afforded to those skilled in the art, as well as a realization of additional advantages thereof, by a consideration of the following detailed description of one or more embodiments. Reference will be made to the appended sheets of drawings that will first be described briefly.

### BRIEF DESCRIPTION OF THE DRAWINGS

Figure 1 schematically illustrates a system for medication delivery, in accordance with one or more embodiments described herein.
Figure 2 schematically illustrates medication packages that may be used in a medication dispensing device, in accordance with one or more embodiments described herein.
Figure 3A-B schematically illustrate a medication dispensing device, in accordance with one or more embodiments described herein.
Figure 4 schematically illustrates a method for medication delivery, in accordance with one or more embodiments described herein.

Embodiments of the present disclosure and their advantages are best understood by referring to the detailed description that follows. It should be appreciated that like reference numerals are used to identify like elements illustrated in one or more of the figures.

### DETAILED DESCRIPTION

A medication dispensing device is typically used to ascertain that the patient takes any prescribed medication at the prescribed time intervals and in the prescribed dose. However, since the medication is often taken in the home of the patient, there is no way of monitoring how the patient is affected by the medication. Some medication may have side-effects such as e.g. edema or dehydration, and there may also be undesirable effects by over- or undermedication. Medication may also be soporific, or exhilarant and cause a change of movement patterns, or cause the body temperature or the blood glucose level to change.

It would be useful to be able to monitor the effects of the medication, and to adjust the medication based on these effects, also in a medication dispensing device that delivers the medication in medication packages. The present disclosure relates generally to systems and methods for medication delivery. Embodiments of the disclosed solution are presented in more detail in connection with the figures.

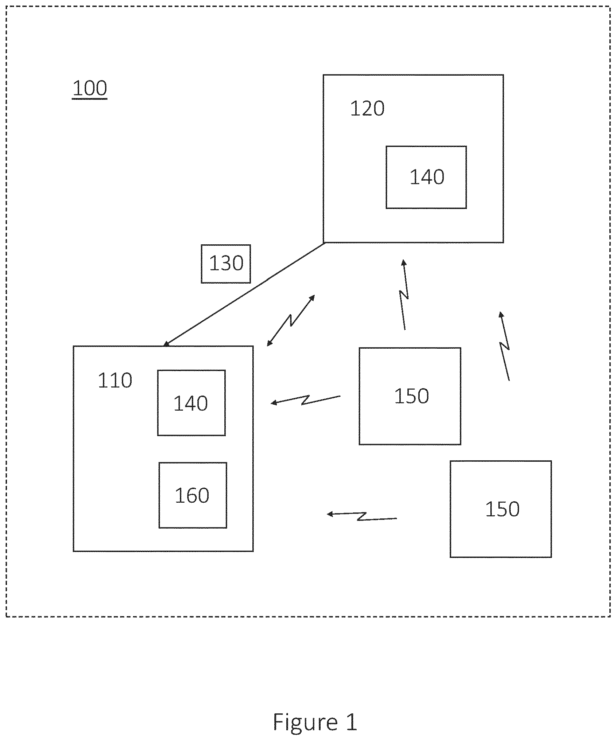

Fig. 1 schematically illustrates a system 100 for medication delivery, in accordance with one or more embodiments described herein. The system 100 schematically illustrated in figure 1 comprises a medication dispensing device 110, a medication distribution system 120, two processors 140, and two sensing arrangements 150. The medication dispensing device 110 may comprise a display 160. The medication dispensing device 110 may be arranged to dispense a medication package 130 containing a predefined dose of medication to a patient at a preset time. The medication distribution system 120 may be arranged to distribute the medication packages 130 containing the predefined doses of medication to the medication dispensing device 110. The at least one sensing arrangement 150 may be arranged to measure at least one patient specific parameter for said patient. The at least one processor 140 may be arranged to process the at least one patient specific parameter, and notify the medication distribution system 120 if the at least one patient specific parameter is determined to deviate from an expected range. The medication distribution system 120 may be arranged to, based on the result of the processing of the at least one patient specific parameter, adjust the dose of medication in the medication packages 130 that are distributed to the medication dispensing device 110. Such a system 100 allows the adjustment of the medication based on the effects of the medication, for a medication dispensing device 110 that delivers the medication in medication packages 130.

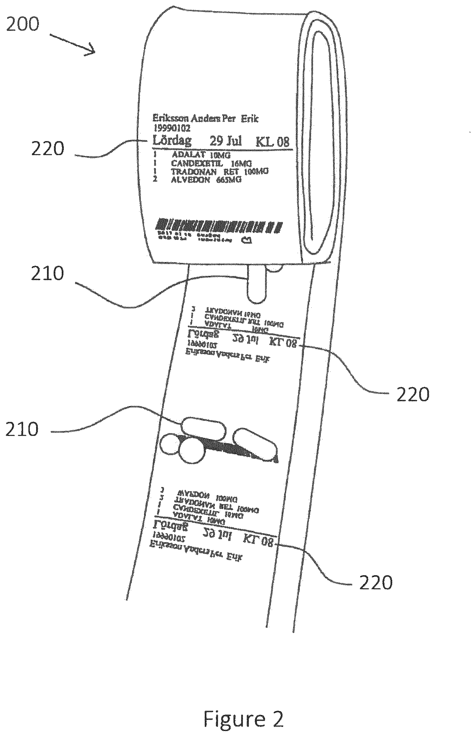

In the embodiment illustrated in Fig. 2, the medication packages 130 come in prepackaged rolls 200, each roll 200 containing a number of medication packages 130 that are attached to each other. Each medication package 130 comprises medication 210, and preferably also a label 220 that may be read by the medication dispensing device 110. The label 220 comprises information about at what time the medication package should be dispensed by the medication dispensing device 110, in a format that may be understood by the medication dispensing device 110, e.g. a barcode or a QR code. The label 220 may also comprise information in a format that may be read by a caregiver before loading the roll 200 into the medication dispensing device 110, and by the patient before taking the medication. This increases the safety of the system, since the caregiver and/or the patient may double-check that the medication is intended for this particular patient.

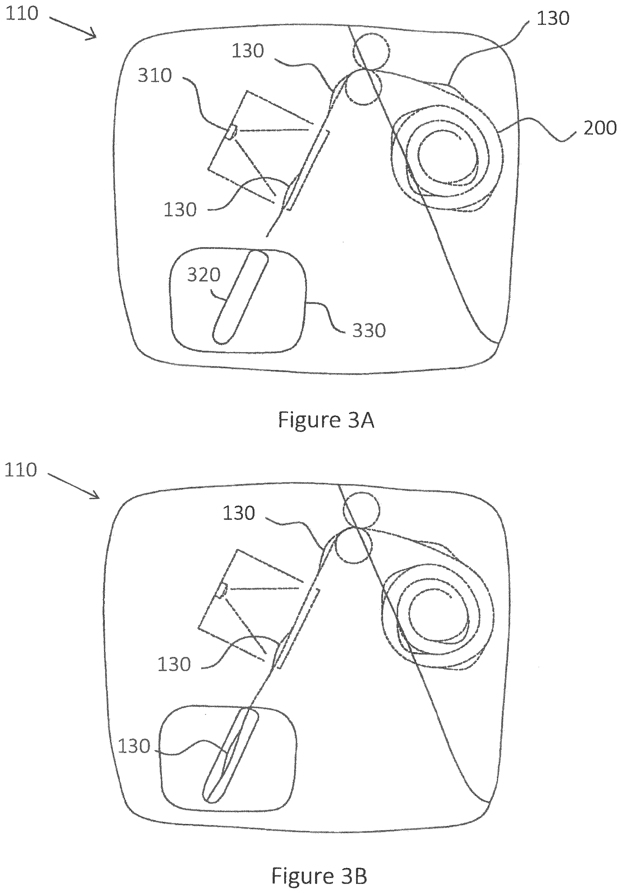

Fig. 3A-B schematically illustrate a medication dispensing device 110 that has been loaded with a roll 200 of medication packages 130. The medication dispensing device 110 preferably comprises a reader 310 that is arranged for reading the information on the label 220 of the next medication package 130 to be dispensed, so that the medication dispensing device 110, based on this information, can dispense the medication package 130 at the correct time to the outlet 330 of the medication dispensing device 110. The medication dispensing device 110 preferably also comprises a sensor 320 that is arranged to sense whether a medication package 130 that has been dispensed has also been retrieved from the outlet 330. In Fig.3A, no medication package 130 has yet been dispensed to the patient, while in Fig. 3B, there is a medication package 130 in the outlet 330, i.e. it has been dispensed but not yet retrieved by the patient.

The advantage of a medication dispensing device 110 such as the one illustrated in Figs. 3A-B, that is loaded with prepackaged rolls 200 of medication packages 130 that comprise labels 220 comprising information about at what time the medication package should be dispensed by the medication dispensing device 110, is that a caregiver in the premises only needs to load the roll 200 into the medication dispensing device 110. Such a medication dispensing device 110 requires no sorting of the right amount of pills into compartments, or any programming of a time schedule for the dispensing of the medication. All of this is taken care of by the medication distribution system 120. There is therefore no need for a medically trained caregiver, such as a doctor or a licensed nurse, for the loading of the medication dispensing device 110.

However, the problem with medication dispensing devices 110 such as the one illustrated in Figs. 3A-B is that since there is no need for a medically trained caregiver for the loading of the medication dispensing device 110, there is a risk that no medically trained caregiver regularly attends to the patient. This increases the risk that side-effects of the medication go unnoticed, and that the dose of medication is therefore not adjusted.

The claimed invention addresses this problem by using at least one sensing arrangement 150 for measuring at least one patient specific parameter for the patient. This parameter is then processed, and if it deviates from an expected range, the medication distribution system 120 is notified. The range may e.g. be set by the physician who has prescribed the medication, but it may also be based on historical data for the patient. The historical data may e.g. be analyzed using a machine learning system, that may automatically determine if the parameter deviates from what is to be expected based on the historical data.

If a deviation has been determined, it may be desirable to adjust the medication. Since the medication is delivered to the patient in prepackaged medication packages 130, the adjustment cannot be done in the premises where the medication dispensing device 110 is located. Instead, an adjustment of the medication requires an adjustment of the content of the medication packages 130 when they are prepackaged. The adjustment therefore needs to be done by the medication distribution system 120.

The medication distribution system 120 comprises a medication storage facility 180 where medication is prepackaged into medication packages 130 that are dispensed by the medication dispensing device 110. The medication packages 130 may be distributed from the medication storage facility 180 to the patient in the same way as medication is normally distributed to the patient, such as e.g. via a pharmacy, where the medication packages 130 may be picked up by a caregiver and delivered to the medication dispensing device 110.

The adjustment of the dose of medication that is distributed to the medication dispensing device 110 based on the at least one patient specific parameter comprises adjusting the content of the medication packages 130. Such an adjustment may e.g. be done based on input from at least one care provider who is responsible for the care of the patient, such as e.g. the physician who has prescribed the medication. This allows the physician who has prescribed the medication to adjust the dose based on the effects of the medication.

The medication distribution system 120 preferably comprises a user interface for the responsible care provider, where the responsible care provider may analyze the at least one patient specific parameter and adjust the prescribed medication accordingly. The responsible care provider may use this user interface to access all the data collected in relation to the one or more patient specific parameters of a patient, regardless of whether the parameters deviate from an expected range. The responsible care provider may in this way closely monitor the status of each individual patient every day, even though the patient stays at home or in a nursing home, in order to determine whether the patient needs extra care. This may be very important for patients with certain illnesses. Since the one or more patient specific parameters are measured automatically, this allows more close care of the patient without the need for a medically trained caregiver to regularly attend to the patient.

In embodiments, the system provides the responsible care provider with a proposal for adjusting the medication, so that the proposal may simply be approved or rejected, or approved after amendments. When an adjustment of the prescribed medication has been approved by the responsible care provider, the medication distribution system 120 distributes new medication packages 130 from the medication storage facility 180 to the medication dispensing device 110 according to the adjusted prescribed medication.

The system 100 may thus be arranged to receive input from at least one care provider who is responsible for the care of the patient regarding the adjustment of the dose of medication that is distributed to the medication dispensing device 110 by the medication distribution system 120. Such a system 100 allows the adjustment of the medication based on the effects of the medication, in a medication dispensing device 110 that delivers the medication in prepackaged medication packages 130.

The system 100 may also comprise a display 160 that may be arranged to display information to the patient. As illustrated in Fig. 1, medication dispensing devices 110 sometimes comprise a display 160 that is e.g. used to display information from the label 220. Such a display 160 may also be used to also display information from the at least one sensing arrangement 150, preferably after some processing. This may be a way to provide feedback to the patient about the effects of the medication.

The display 160 may, as explained, be arranged on the medication dispensing device 110. However, a display 160 may also, or additionally, be arranged elsewhere, such as e.g. on a device that is wearable by the patient, such as e.g. a watch or a wristband. The display on a smartphone may also, or additionally, be used as the display 160 of the system 100.

The display 160 may be used for communication between the patient and the responsible care provider, e.g. in the form of video conferencing. The system 100 may also comprise a microphone and a loudspeaker, in order to enable such video conferencing. The display 160, the microphone and the loudspeaker may e.g. be arranged on the medication dispensing device 110. However, it is also possible to use the display 160, the microphone and the loudspeaker of a smartphone.

As illustrated in Fig. 1, the system 100 may comprise one or more processors 140, and the processors 140 may be located in the medication dispensing device 110, in the medication distribution system 120, or elsewhere, such as e.g. in the at least one sensing arrangement 150. The at least one sensing arrangement 150 may transfer the at least one patient specific parameter, in raw format or processed, to the at least one processor 140, and thus either to the medication dispensing device 110, to the medication distribution system 120, or both.

The at least one sensing arrangement 150 is preferably arranged in the same premises as the medication dispensing device 110. If the medication dispensing device 110 is arranged in the patient's home, the at least one sensing arrangement 150 should also be arranged in the patient's home. The premises are however not necessarily the home of the patient, they may also be e.g. a part of a nursing home. The at least one sensing arrangement 150 may e.g. be arranged in a position where the patient is located when receiving medication from the medication dispensing device 110. This ensures that the at least one patient specific parameter is measured at least at the times that the patient receives medication.

The at least one sensing arrangement 150 may comprise: a body weight sensor, a motion sensor, a temperature sensor, proximity sensor, a temperature sensor, a continence sensor, a breathing frequency sensor, a heart rate sensor, a blood pressure sensor, an oxygen saturation sensor, and/or a blood glucose sensor. All these sensors may provide information regarding how the patient is affected by the medication.

Such a sensing arrangement may be used to gather patient specific data continuously, in periods, in connection with certain activities of the patient or at certain occasions, possibly prompted by a signal from the medication dispensing device 110. Sensor data may be communicated to the medication dispensing device 110 and/or to the medication distribution system 120. The sensor data may be processed to detect a selection of a sensor data pattern that for example deviates from a normal pattern of the patient, a predicted pattern of the patient due to medication, dose of medication, the time of taking the medication, or other factors. The use of historical data for determining deviations from expected values, based on e.g. daily mean values or the pattern of parameter fluctuation over time, e.g. using a machine learning system, may thus apply to all the patient specific parameters that may be collected by such sensors. Thus, historical data from the at least one sensing arrangement 150 may be used for determining whether the at least one patient specific parameter deviates from the expected range, e.g. using a machine learning system, based on e.g. daily mean values and/or the pattern of parameter fluctuation over time. Machine learning is an efficient way of analyzing large quantities of data.

The at least one sensing arrangement 150 may comprise a fingertip sensor, such as e.g. a pulse oximeter. It is possible to measure a number of patient specific parameters, such as e.g. body temperature, heart rate, oxygen saturation, and blood glucose level, non-invasively, by cutaneous measurements. The medication dispensing device 110 may be controlled to only dispense the medication package 130 to the patient if the patient puts a fingertip in the fingertip sensor. This ensures that the at least one patient specific parameter is measured at least at the times when the patient receives medication. The fingertip sensor may e.g. be integrated into the medication dispensing device 110.

The at least one sensing arrangement 150 may comprise a body weight measuring device, that may e.g. be arranged in the form of weight sensors provided on or in an object on which the weight of the patient regularly rests, such as e.g. a carpet or rug, a mattress, a chair, a sofa, an armchair, or a toilet seat. This ensures that the current weight of the patient is determined on a regular basis without any action being required from the patient. A carpet or rug may e.g. be arranged on the floor where the patient needs to be standing in order to be able to receive medication from the medication dispensing device 110.

A body weight measuring device arranged in the form of a carpet or rug provided with weight sensors may also have other uses. Patients suffering from dementia type illnesses often need to be monitored so that an alarm can be sent if they behave in unexpected ways. A number of different carpets or rugs provided with weight sensors that are arranged in the home of such a patient may be used to track the movement of such a patient. Based on historical data regarding previous movement, it can be determined whether the movement pattern has changed, e.g. using machine learning based on the historical data. The system may be able to determine that there is a high likelihood that it is actually this particular patient that is standing on the body weight measuring device based on the previous movement of the patient.

Such movement tracking can also be used to alert a caregiver if the patient moves beyond a predetermined area, such as e.g. the area within a nursing home that this particular patient is allowed to access, and also e.g. track if the patient is leaving the home. Even if many different ways of tracking patients suffering from dementia type illnesses are already known, they normally rely on tracking devices arranged on the patient, such as in the form of a wristband or a necklace. Patients suffering from dementia type illnesses are however prone to removing any such tracking devices, so a system that is independent of any such equipment would be more secure.

The at least one sensing arrangement 150 may e.g. be arranged to monitor any changes in body weight of the patient over time, e.g. by calculating a daily mean body weight and monitor the change in said daily mean body weight over time, and automatically notifying the medication distribution system 120 if the at least one patient specific parameter is determined to deviate from an expected range. In the same way as for the movement pattern, historical data regarding the pattern of weight fluctuation during the day may also be fed into a machine learning system, that based on this may be able to automatically detect whether the pattern of weight fluctuation during the day suddenly changes compared to the historical data.

If a patient gains weight, or loses weight, this may be an indication of an impaired health status. Monitoring the body weight of a patient on a regular basis is therefore one method of indicating changes of the health status of the patient early. For some types of medication, a weight gain or loss may be expected. Changes of the dosage of a medication may also result in an expected weight gain or loss. Information regarding patient specific types of medication, and the dosages thereof, may be stored and made available in the system 100. The specification of each medication package 130 that has been output from the medication dispensing device 110 to a patient may be stored in a local memory of the medication dispensing device 110 or elsewhere in the system 100. The results from a weight monitoring process may for such cases be compared to a predicted and pre-stored development of an expected weight gain or loss. Thereby, early indications of changes to the health status of the patient is provided.

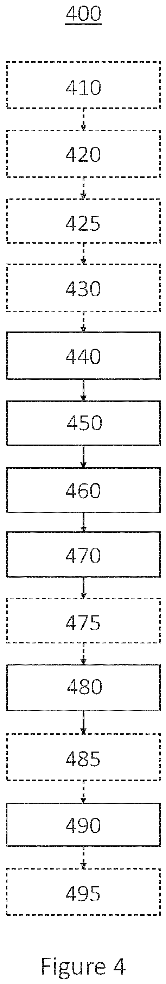

Figure 4 schematically illustrates a method 400 for medication delivery, in accordance with one or more embodiments described herein. The method 400 may comprise:
Step 440: automatically delivering a medication package 130 containing a predefined dose of medication to a patient at a preset time, using a medication dispensing device 110.

Step 450: automatically distributing the medication packages 130 containing the predefined doses of medication to the medication dispensing device 110, using a medication distribution system 120.

Step 460: automatically measuring at least one patient specific parameter for the patient using at least one sensing arrangement 150.

Step 470: automatically processing, in at least one processor 140, the at least one patient specific parameter.

Step 480: automatically notifying the medication distribution system 120 if the at least one patient specific parameter is determined to deviate from an expected range.

Step 490: adjusting, based on the result of the processing of the at least one patient specific parameter, the dose of medication in the medication packages 130 that are distributed by the medication distribution system 120 to the medication dispensing device 110.

Such a method allows the adjustment of the medication based on the effects of the medication, for a medication dispensing device 110 that delivers the medication in medication packages 130.

In embodiments, the processing 470 comprises using historical data from the at least one sensing arrangement 150 for determining whether the at least one patient specific parameter deviates from the expected range, e.g. using a machine learning system, based on e.g. daily mean values and/or the pattern of parameter fluctuation over time. Machine learning is an efficient way of analyzing large quantities of data.

The method 400 may further comprise one or more of:
Step 410: arranging the at least one sensing arrangement 150 in a position where the patient is located when receiving medication from the medication dispensing device. This ensures that the at least one patient specific parameter is measured at least at the times that the patient receives medication.

Step 420: arranging the at least one sensing arrangement 150 to comprise a body weight measuring device.

Step 425: arranging the at least one sensing arrangement 150 to comprise: a body weight sensor, a motion sensor, a temperature sensor, proximity sensor, a temperature sensor, a continence sensor, a breathing frequency sensor, a heart rate sensor, a blood pressure sensor, and/or a blood glucose sensor. All these sensors may provide information regarding how the patient is affected by the medication.

Step 430: arranging the body weight measuring device in the form of weight sensors provided on or in an object on which the weight of the patient regularly rests, such as e.g. a carpet or rug, a mattress, a chair, a sofa, an armchair, or a toilet seat provided with weight sensors. This ensures that the current weight of the patient is determined on a regular basis without any action being required from the patient.

Step 475: automatically monitoring any changes in body weight of the patient over time, e.g. by calculating a daily mean body weight and monitor any change in said daily mean body weight over time. In order for a determination of a daily mean body weight to be meaningful, the body weight should be measured a number of times each day, e.g. at least three times a day.

Step 485: receiving input from at least one care provider who is responsible for the care of the patient regarding the adjustment of the dose of medication that is distributed to the medication dispensing device 110 by the medication distribution system 120. This allows the physician who has prescribed the medication to adjust the dose based on the effects of the medication.

Step 495: displaying information about the result of the processing of the at least one patient specific parameter to the patient on a display 160. This may be a way to provide feedback to the patient about the effects of the medication. Such a display 160 may be arranged on the medication dispensing device 110, but it may also be integrated into a device that e.g. is wearable by the patient, such as e.g. a watch or a wristband. It is also possible to use the display 160 on e.g. a smartphone.

## Claims

1. System for (100) for medication delivery, the system (100) comprising:
a medication dispensing device (110), arranged to deliver a medication package (130) containing a predefined dose of medication to a patient at a preset time;
a medication distribution system (120), arranged to distribute said medication packages (130) containing said predefined dose of medication to the medication dispensing device (110);
at least one sensing arrangement (150), arranged in the same premises as the medication dispensing device (110), and arranged to measure at least one patient specific parameter for said patient; and
at least one processor (140), arranged to process the at least one patient specific parameter, and notify the medication distribution system (120) if the at least one patient specific parameter is determined to deviate from an expected range,
wherein the medication distribution system (120) comprises a medication storage facility (180) where said predefined dose of medication is prepackaged into said medication packages (130), and is arranged to, based on the result of the processing of the at least one patient specific parameter, adjust the predefined dose of medication that is prepackaged into said medication packages (130).

2. System (100) according to claim 1, wherein the medication distribution system (120) is arranged to receive input from at least one care provider who is responsible for the care of the patient regarding the adjustment of the dose of medication in said medication packages (130).

3. System (100) according to claim 1 or 2, wherein the system (100) comprises a display (160), which is arranged to display information about the result of the processing of the at least one patient specific parameter to the patient.

4. System (100) according to claim 3, wherein the display (160) is integrated into a device that is wearable by the patient, such as e.g. a watch or a wristband.

5. System (100) according to any one of claims 1-4, wherein the at least one sensing arrangement (150) is arranged in a position where the patient is located when receiving medication from the medication dispensing device (110).

6. System (100) according to any one of claims 1-5, wherein the at least one sensing arrangement (150) comprises a body weight measuring device.

7. System (100) according to claim 6, wherein the body weight measuring device is arranged in the form of weight sensors provided on or in an object on which the weight of the patient regularly rests, such as e.g. a carpet or rug, a mattress, a chair, a sofa, an armchair, or a toilet seat.

8. System (100) according to any one of claims 1-7, wherein the at least one sensing arrangement (150) comprises: a body weight sensor, a motion sensor, a temperature sensor, a proximity sensor, a temperature sensor, a continence sensor, a breathing frequency sensor, a heart rate sensor, a blood pressure sensor, and/or a blood glucose sensor.

9. Method (400) for medication delivery by using the system of claim 1, the method (400) comprising:
automatically delivering (440) a medication package (130) containing a predefined dose of medication to a patient at a preset time, using a medication dispensing device (110);
automatically distributing (450) said medication packages (130) containing said predefined dose of medication to the medication dispensing device (110), using a medication distribution system (120) comprising a medication storage facility (180), where said predefined dose of medication is prepackaged into said medication packages (130), and;
automatically measuring (460) at least one patient specific parameter for said patient using at least one sensing arrangement (150), arranged in the same premises as the medication dispensing device (110);
automatically processing (470), in at least one processor (140), the at least one patient specific parameter;
automatically notifying (480) the medication distribution system (120) if the at least one patient specific parameter is determined to deviate from an expected range; and
adjusting (490), based on the result of the processing of the at least one patient specific parameter, the predefined dose of medication that is prepackaged into said medication packages (130).

10. Method (400) according to claim 9, further comprising receiving (485) input from at least one care provider who is responsible for the care of the patient regarding the adjustment of the dose of medication in said medication packages (130).

11. Method (400) according to claim 9 or 10, further comprising displaying (495) information about the result of the processing of the at least one patient specific parameter to the patient on a display (160).

12. Method (400) according to any one of claims 9-11, further comprising arranging (420) the at least one sensing arrangement (150) to comprise a body weight measuring device, and arranging (430) the body weight measuring device in the form of weight sensors provided on or in an object on which the weight of the patient regularly rests, such as e.g. a carpet or rug, a mattress, a chair, a sofa, an armchair, or a toilet seat.

13. Method (400) according to any one of claims 9-12, further comprising automatically monitoring (475) any changes in body weight of the patient over time, e.g. by calculating a daily mean body weight and monitor any change in said daily mean body weight over time.

14. Method (400) according to any one of claims 9-13, further comprising arranging (425) the at least one sensing arrangement (150) to comprise: a body weight sensor, a motion sensor, a temperature sensor, proximity sensor, a temperature sensor, a continence sensor, a breathing frequency sensor, a heart rate sensor, a blood pressure sensor, and/or a blood glucose sensor.

15. Method (400) according to any one of claims 9-14, wherein the processing (470) comprises using historical data from the at least one sensing arrangement (150) for determining whether the at least one patient specific parameter deviates from the expected range, e.g. using a machine learning system, based on e.g. daily mean values and/or the pattern of parameter fluctuation over time.

## Patentansprüche

1. System (100) zur Verabreichung von Medikamenten, das System (100) umfassend:
eine Medikamentenabgabevorrichtung (110), die angeordnet ist, um eine Medikamentenverpackung (130), die eine vordefinierte Medikamentendosis enthält, zu einem vorgegebenen Zeitpunkt an einen Patienten abzugeben;
ein Medikamentenverteilungssystem (120), das angeordnet ist, um die Medikamentenverpackungen (130), die die vordefinierte Medikamentendosis enthalten, an die Medikamentenabgabevorrichtung (110) zu verteilen;
mindestens eine Sensoranordnung (150), die in denselben Räumlichkeiten wie die Medikamentenabgabevorrichtung (110) angeordnet ist und mindestens einen patientenspezifischen Parameter für den Patienten messen kann; und
mindestens einen Prozessor (140), der angeordnet ist, um den mindestens einen patientenspezifischen Parameter zu verarbeiten und das Medikamentenverteilungssystem (120) zu benachrichtigen, wenn bestimmt wird, dass der mindestens eine patientenspezifische Parameter von einem erwarteten Bereich abweicht,
wobei das Medikamentenverteilungssystem (120) einen Medikamentenspeicher (180) umfasst, in dem die vordefinierte Medikamentendosis in die Medikamentenverpackungen (130) vorverpackt wird, und angeordnet ist, um basierend auf dem Ergebnis der Verarbeitung des mindestens einen patientenspezifischen Parameters die vordefinierte Medikamentendosis, die in die Medikamentenverpackungen (130) vorverpackt wird, anzupassen.

2. System (100) nach Anspruch 1, wobei das Medikamentenverteilungssystem (120) angeordnet ist, um Eingaben von mindestens einem Pflegeanbieter, der für die Pflege des Patienten verantwortlich ist, bezüglich der Einstellung der Medikamentendosis in den Medikamentenverpackungen (130) zu empfangen.

3. System (100) nach Anspruch 1 oder 2, wobei das System (100) eine Anzeige (160) umfasst, das angeordnet ist, um dem Patienten Informationen über das Ergebnis der Verarbeitung des mindestens einen patientenspezifischen Parameters anzuzeigen.

4. System (100) nach Anspruch 3, wobei die Anzeige (160) in eine Vorrichtung integriert ist, die vom Patienten getragen werden kann, wie beispielsweise eine Uhr oder ein Armband.

5. System (100) nach einem der Ansprüche 1 bis 4, wobei die mindestens eine Erfassungsanordnung (150) an einer Position angeordnet ist, an der sich der Patient befindet, wenn er Medikamente von der Medikamentenabgabevorrichtung (110) empfängt.

6. System (100) nach einem der Ansprüche 1 bis 5, wobei die mindestens eine Sensoranordnung (150) eine Körpergewichtsmessvorrichtung umfasst.

7. System (100) nach Anspruch 6, wobei die Körpergewichtsmessvorrichtung in Form von Gewichtssensoren angeordnet ist, die auf oder in einem Gegenstand bereitgestellt werden, auf dem das Gewicht des Patienten regelmäßig ruht, wie beispielsweise einem Teppich oder Vorleger, einer Matratze, einem Stuhl, einem Sofa, einem Sessel oder einem Toilettensitz.

8. System (100) nach einem der Ansprüche 1 bis 7, wobei die mindestens eine Sensoranordnung (150) Folgendes umfasst: einen Körpergewichtssensor, einen Bewegungssensor, einen Temperatursensor, einen Näherungssensor, einen Temperatursensor, einen Kontinenzsensor, einen Atemfrequenzsensor, einen Herzfrequenzsensor, einen Blutdrucksensor und/oder einen Blutzuckersensor.

9. Verfahren (400) zur Medikamentenabgabe unter Verwendung des Systems nach Anspruch 1, das Verfahren (400) umfassend:
automatisches Abgeben (440) einer Medikamentenverpackung (130), die eine vordefinierte Medikamentendosis enthält, an einen Patienten zu einem voreingestellten Zeitpunkt, unter Verwendung einer Medikamentenabgabevorrichtung (110);
automatisches Verteilen (450) der Medikamentenverpackungen (130), die die vordefinierte Medikamentendosis enthalten, an die Medikamentenabgabevorrichtung (110) unter Verwendung eines Medikamentenverteilungssystems (120), das einen Medikamentenspeicher (180) umfasst, wo die vordefinierte Medikamentendosis in die Medikamentenverpackungen (130) vorverpackt wird, und;
automatisches Messen (460) mindestens eines patientenspezifischen Parameters für den Patienten unter Verwendung mindestens einer Sensoranordnung (150), die in denselben Räumlichkeiten wie die Medikamentenabgabevorrichtung (110) angeordnet ist;
automatisches Verarbeiten (470), in mindestens einem Prozessor (140), des mindestens einen patientenspezifischen Parameters;
automatisches Benachrichtigen (480) des Medikamentenverteilungssystems (120), wenn bestimmt wird, dass der mindestens eine patientenspezifische Parameter von einem erwarteten Bereich abweicht; und Anpassen (490), basierend auf dem Ergebnis der Verarbeitung des mindestens einen patientenspezifischen Parameters, der vordefinierten Medikamentendosis, die in den Medikamentenverpackungen (130) vorverpackt ist.

10. Verfahren (400) nach Anspruch 9, ferner umfassend das Empfangen (485) von Eingaben von mindestens einem Pflegeanbieter, der für die Pflege des Patienten verantwortlich ist, bezüglich der Anpassung der Medikamentendosis in den Medikamentenverpackungen (130).

11. Verfahren (400) nach Anspruch 9 oder 10, ferner umfassend das Anzeigen (495) von Informationen über das Ergebnis der Verarbeitung des mindestens einen patientenspezifischen Parameters an den Patienten auf einer Anzeige (160).

12. Verfahren (400) nach einem der Ansprüche 9 bis 11, ferner umfassend das Anordnen (420) der mindestens einen Sensoranordnung (150), um eine Körpergewichtsmessvorrichtung bereitzustellen, und das Anordnen (430) der Körpergewichtsmessvorrichtung in Form von Gewichtssensoren, die auf oder in einem Gegenstand vorgesehen sind, auf dem das Gewicht des Patienten regelmäßig ruht, wie beispielsweise einem Teppich oder Vorleger, einer Matratze, einem Stuhl, einem Sofa, einem Sessel oder einem Toilettensitz.

13. Verfahren (400) nach einem der Ansprüche 9 bis 12, ferner umfassend das automatische Überwachen (475) jeglicher Veränderungen des Körpergewichts des Patienten im Laufe der Zeit, z. B. durch Berechnen eines täglichen mittleren Körpergewichts und Überwachen jeglicher Veränderung des täglichen mittleren Körpergewichts im Laufe der Zeit.

14. Verfahren (400) nach einem der Ansprüche 9 bis 13, ferner umfassend das Anordnen (425) der mindestens einen Sensoranordnung (150), um Folgendes zu umfassen: einen Körpergewichtssensor, einen Bewegungssensor, einen Temperatursensor, einen Näherungssensor, einen Temperatursensor, einen Kontinenzsensor, einen Atemfrequenzsensor, einen Herzfrequenzsensor, einen Blutdrucksensor und/oder einen Blutzuckersensor.

15. Verfahren (400) nach einem der Ansprüche 9 bis 14, wobei die Verarbeitung (470) die Verwendung historischer Daten von der mindestens einen Sensoranordnung (150) umfasst, um zu bestimmen, ob der mindestens eine patientenspezifische Parameter von dem erwarteten Bereich abweicht, z. B. unter Verwendung eines maschinellen Lernsystems, basierend auf z. B. täglichen Mittelwerten und/oder dem Muster der Parameterfluktuation über die Zeit.

## Revendications

1. Système (100) de distribution de médicaments, le système (100) comprenant :
un dispositif (110) d'administration de médicament, agencé pour délivrer un emballage (130) de médicament contenant une dose prédéfinie de médicament à un patient à un moment prédéfini ;
un système (120) de distribution de médicament, agencé pour distribuer lesdits emballages (130) de médicament contenant ladite dose prédéfinie de médicament au dispositif (110) d'administration de médicament ;
au moins un dispositif (150) de détection, agencé dans les mêmes locaux que le dispositif (110) d'administration de médicament, et agencé pour mesurer au moins un paramètre particulier au patient pour ledit patient ; et
au moins un processeur (140), agencé pour traiter l'au moins un paramètre particulier au patient, et notifier le système (120) de distribution de médicament si l'au moins un paramètre particulier au patient est déterminé pour dévier d'une plage attendue,
dans lequel le système (120) de distribution de médicament comprend une installation (180) de stockage de médicament dans laquelle ladite dose prédéfinie de médicament est préemballée dans lesdits emballages (130) de médicament, et est agencé pour, sur la base du résultat du traitement de l'au moins un paramètre particulier au patient, ajuster la dose prédéfinie de médicament qui est préemballée dans lesdits emballages (130) de médicament.

2. Système (100) selon la revendication 1, dans lequel le système (120) de distribution de médicament est agencé pour recevoir une entrée d'au moins un prestataire de soins qui est responsable des soins du patient concernant l'ajustement de la dose de médicament dans lesdits emballages (130) de médicament.

3. Système (100) selon la revendication 1 ou la revendication 2, dans lequel le système (100) comprend un affichage (160), qui est agencé pour afficher des informations sur le résultat du traitement au patient de l'au moins un paramètre particulier au patient.

4. Système (100) selon la revendication 3, dans lequel l'affichage (160) est intégré dans un dispositif qui peut être porté par le patient, tel qu'une montre ou un bracelet.

5. Système (100) selon l'une quelconque des revendications 1 à 4, dans lequel l'au moins un dispositif (150) de détection est agencé dans une position où le patient est situé lors de la réception d'un médicament à partir du dispositif (110) d'administration de médicament.

6. Système (100) selon l'une quelconque des revendications 1 à 5, dans lequel l'au moins un dispositif (150) de détection comprend un dispositif de mesure du poids corporel.

7. Système (100) selon la revendication 6, dans lequel le dispositif de mesure du poids corporel est agencé sous la forme de capteurs de poids fournis sur ou dans un objet sur lequel repose régulièrement le poids du patient, tel qu'un tapis, un matelas, une chaise, un canapé, un fauteuil ou un siège de toilettes.

8. Système (100) selon l'une quelconque des revendications 1 à 7, dans lequel l'au moins un dispositif (150) de détection comprend : un capteur de poids corporel, un capteur de mouvement, un capteur de température, un capteur de proximité, un capteur de température, un capteur de continence, un capteur de fréquence respiratoire, un capteur de fréquence cardiaque, un capteur de pression artérielle et/ou un capteur de glycémie.

9. Procédé (400) de distribution de médicament à l'aide du système de la revendication 1, le procédé (400) comprenant :
la distribution (440) automatique d'un emballage (130) de médicament contenant une dose prédéfinie de médicament à un patient à un moment prédéfini, à l'aide d'un dispositif (110) d'administration de médicament ;
la distribution (450) automatique desdits emballages (130) de médicament contenant ladite dose prédéfinie de médicament vers le dispositif (110) d'administration de médicament, à l'aide d'un système (120) de distribution de médicament comprenant une installation (180) de stockage de médicament, où ladite dose prédéfinie de médicament est préemballée dans lesdits emballages (130) de médicament, et ;
la mesure (460) automatique d'au moins un paramètre particulier au patient pour ledit patient en utilisant au moins un dispositif (150) de détection, agencé dans les mêmes locaux que le dispositif (110) d'administration de médicament ;
le traitement (470) automatique, dans au moins un processeur (140), de l'au moins un paramètre particulier au patient ;
la notification (480) automatique au système (120) de distribution de médicament si l'au moins un paramètre particulier de patient est déterminé pour dévier d'une plage attendue ; et
l'ajustement (490), sur la base du résultat du traitement de l'au moins un paramètre particulier au patient, de la dose prédéfinie de médicament qui est préemballée dans lesdits emballages (130) de médicament.

10. Procédé (400) selon la revendication 9, comprenant en outre la réception (485) d'une entrée d'au moins un prestataire de soins qui est responsable des soins du patient concernant l'ajustement de la dose de médicament dans lesdits emballages (130) de médicament.

11. Procédé (400) selon la revendication 9 ou la revendication 10, comprenant en outre l'affichage (495) d'informations sur le résultat du traitement de l'au moins un paramètre particulier au patient sur un affichage (160).

12. Procédé (400) selon l'une quelconque des revendications 9 à 11, comprenant en outre l'agencement (420) de l'au moins un dispositif (150) de détection pour comprendre un dispositif de mesure du poids corporel, et l'agencement (430) du dispositif de mesure du poids corporel sous la forme de capteurs de poids fournis sur ou dans un objet sur lequel repose régulièrement le poids du patient, tel qu'un tapis ou une moquette, un matelas, une chaise, un canapé, un fauteuil ou un siège de toilettes.

13. Procédé (400) selon l'une quelconque des revendications 9 à 12, comprenant en outre la surveillance (475) automatique de toute modification du poids corporel du patient au fil du temps, par exemple en calculant un poids corporel moyen quotidien et en surveillant toute modification dudit poids corporel moyen quotidien au fil du temps.

14. Procédé (400) selon l'une quelconque des revendications 9 à 13, comprenant en outre l'agencement (425) de l'au moins un dispositif (150) de détection pour comprendre : un capteur de poids corporel, un capteur de mouvement, un capteur de température, un capteur de proximité, un capteur de température, un capteur de continence, un capteur de fréquence respiratoire, un capteur de fréquence cardiaque, un capteur de pression artérielle et/ou un capteur de glycémie.

15. Procédé (400) selon l'une quelconque des revendications 9 à 14, dans lequel le traitement (470) comprend l'utilisation de données historiques provenant de l'au moins un dispositif (150) de détection pour déterminer si l'au moins un paramètre spécifique au patient dévie de la plage attendue, par exemple en utilisant un système d'apprentissage automatique, sur la base, par exemple, de valeurs moyennes quotidiennes et/ou du schéma de fluctuation du paramètre dans le temps.
